(19) Europäisches Patentamt
European Patent Office
Office européen des brevets

(11) Veröffentlichungsnummer: **0 159 587**
**A2**

# (12) EUROPÄISCHE PATENTANMELDUNG

(21) Anmeldenummer: **85103981.8**

(22) Anmeldetag: **02.04.85**

(51) Int. Cl.⁴: **C 07 C 127/22**, A 01 N 47/34

(30) Priorität: **25.04.84 DE 3415386**

(43) Veröffentlichungstag der Anmeldung: **30.10.85 Patentblatt 85/44**

(84) Benannte Vertragsstaaten: **BE CH DE FR GB IT LI NL**

(71) Anmelder: **BASF Aktiengesellschaft, Carl-Bosch-Strasse 38, D-6700 Ludwigshafen (DE)**

(72) Erfinder: **Lange, Arno, Dr., Oberes Geistal 3b, D-6702 Bad Duerkheim (DE)**
Erfinder: **Schirmer, Ulrich, Dr., Berghalde 79, D-6900 Heidelberg (DE)**
Erfinder: **Hofmeister, Peter, Dr., Bruesseler Ring 32, D-6700 Ludwigshafen (DE)**
Erfinder: **Adolphi, Heinrich, Dr., Kalmitweg 11, D-6703 Limburgerhof (DE)**

## (54) N-Benzoyl-,N'-stilbenylharnstoffe, Verfahren zu ihrer Herstellung und Verwendung zur Bekämpfung von Schädlingen.

(57) N-Benzoyl-,N'-stilbenylharnstoffe der Formeln I bzw. Ia

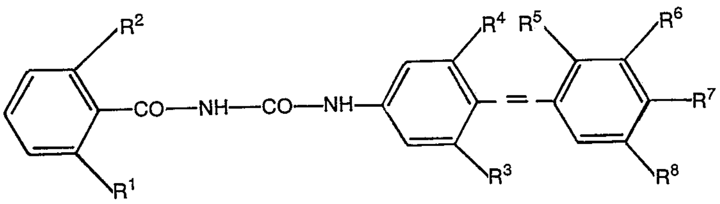

(I),

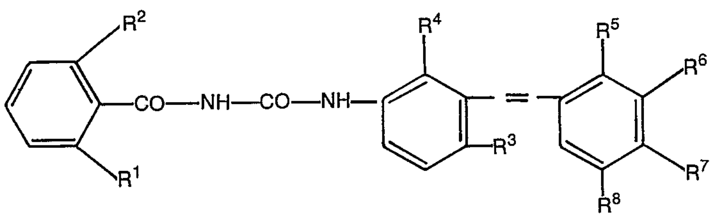

(Ia)

in denen bedeutet
$R^1$ Fluor, Chlor oder Methyl,
$R^2$ Fluor, Chlor oder Wasserstoff,
$R^3$ und $R^4$ gleiche oder verschiedene Substituenten, nämlich Wasserstoff, Chlor, Brom, Fluor oder Trifluormethyl und
$R^5$ bis $R^8$ Wasserstoff oder mindestens einen Substituenten, nämlich Fluor, Chlor, Brom, Alkyl mit 1 bis 4 C-Ato-

EP 0 159 587 A2

men, das mit Fluor und/oder Chlor substituiert sein kann, Alkoxi mit 1 bis 4 C-Atomen, das mit Fluor und/oder Chlor substituiert ist,

ein Verfahren zu ihrer Herstellung sowie ihre Verwendung als Schädlingsbekämpfungsmittel, insbesondere gegen Insekten und Akariden.

N-Benzoyl-,N'-stilbenylharnstoffe, Verfahren zu ihrer Herstellung und Verwendung zur Bekämpfung von Schädlingen

Die Erfindung betrifft N-Benzoyl-,N'-stilbenylharnstoffe, Verfahren zu ihrer Herstellung und Schädlingsbekämpfungsmittel, die diese Verbindungen als Wirkstoffe enthalten.

Es ist bekannt, daß Wirkstoffe auf der Grundlage von N-Benzoyl-,N'-phenylharnstoffen als Insektizide verwendet werden können (J. Agr.Food Chem.21, 348 (1973)).

Die bekannten Wirkstoffe sind zur Bekämpfung zum Beispiel von Blattläusen und von Fliegen relativ wenig geeignet, so daß Bedarf besteht für neue N-Benzoyl-N'-phenylharnstoffe mit erweiterter oder anderer Wirkungsrichtung.

Es wurde nun gefunden, daß N-Benzoyl-,N'-stilbenylharnstoffe diese Eigenschaft in besonderer Weise besitzen und gegen verschiedene schwer bekämpfbarer oder mit bekannten N-Benzoyl-N'-phenylharnstoffen schwer bekämpfbarer Arten sehr gut verwendet werden können. Die N-Benzoyl-N'-stilbenylharnstoffe können in verschiedener Weise substituiert sein und lassen sich durch die allgemeinen Formeln (I) bzw. (Ia) wiedergeben

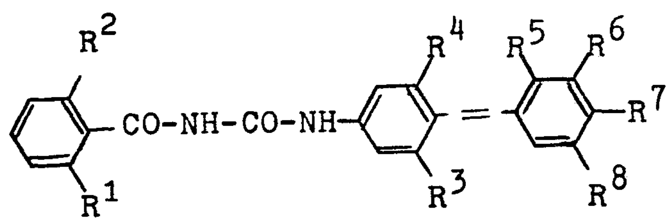

(I),

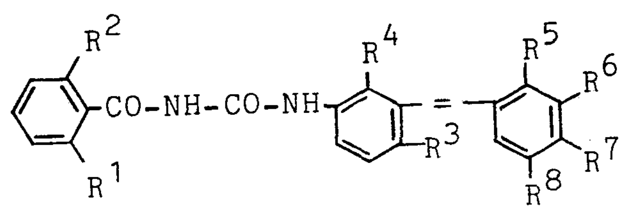

(Ia)

in denen bedeutet:

$R^1$ Fluor, Chlor oder Methyl

$R^2$ Fluor, Chlor oder Wasserstoff

$R^3$ und $R^4$ gleiche oder verschiedene Substituenten, nämlich Wasserstoff, Chlor, Brom, Fluor oder Trifluormethyl und

$R^5$ bis $R^8$ Wasserstoff oder mindestens einen Substituenten, nämlich Fluor, Chlor, Brom, Alkyl mit 1 - 4 C-Atomen, das mit Fluor und/oder Chlor substituiert sein kann, Alkoxi mit 1 - 4 C-Atomen, das ein- oder mehrfach mit Fluor und/oder Chlor substituiert ist.

Wie der Vergleich von Formel I mit Ia zeigt, kann die Verknüpfungsstelle des Stilbenzylrestes mit dem Harnstoffrest sich in meta- oder para-Posititon zur Doppelbindung befinden. Die Verbindungen können sich von cis- oder von trans-Stilben ableiten.

Bevorzugt sind Verbindungen, bei denen $R^1 = R^2 = F$ oder $R^1 = F$ und $R^2 = Cl$ oder $R^1 = Cl$ und $R^2 = H$ oder $R^1 = R^2 = Cl$ ist.

Verbindungen der Formel I sind gegenüber solchen der Formel Ia ebenfalls bevorzugt.

Weiter bevorzugt sind Verbindungen, bei denen $R^3$ Chlor oder Fluor ist.

Besonders wirksam sind Verbindungen, bei denen nur einer oder zwei der Reste $R^5$ bis $R^8$ von Wasserstoff verschieden ist und in diesem Falle aus der Gruppe F, Cl, Br, $OCHF_2$, $OCF_3$, $OC_2HF_4$, $CF_3$ gewählt ist.

Man erhält die erfindungsgemäßen N-Benzoyl-,N'-stilbenylharnstoffe auf jede Weise die zur Herstellung der entsprechenden bekannten Verbindungen verwendet werden kann. Beispielsweise kann man eine entsprechende Verbindung der Formel II

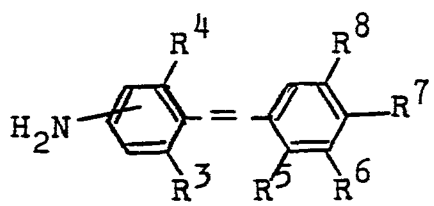

(II),

mit einen entsprechenden Benzoylisocyanat der Formel III

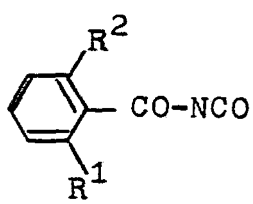

(III),

oder, falls die benötigten Ausgansstoffe II bzw. III nicht verfügbar sind oder zu teuer einstehen, eine entsprechende Verbindung der Formel IV

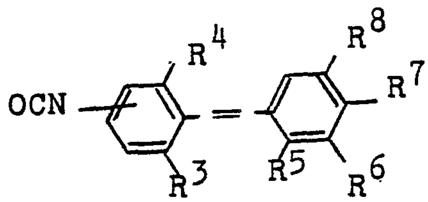 (IV),

mit einem entsprechenden Benzamid der Formel V

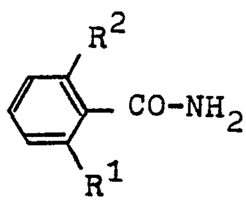 (V),

umsetzen.

Umsetzungen dieser Art, geeignete Bedingungen, Lösungsmittel und andere Hinweise sind z.B. beschrieben in der DE-AS 21 23 236.

Die Umsetzungen verlaufen teilweise quantitativ und liefern meist von vornherein einheitliche Produkte, gegebenenfalls können die üblichen Methoden zur Reinigung, z.B. Umkristallisieren angewandt werden. Zu ihrer Charakterisierung dienen Elementaranalyse, Schmelzpunkt, IR- und NMR-Spektrum.

Zur Durchführung des erstgenannten Verfahrens wird zweckmäßigerweise das substituierte Anilin (Aminostilben II) zusammen mit einem Lösungs- oder Verdünnungsmittel vorgelegt und eine stöchiometrische Menge an Isocyanat (III) zugegeben. Die Umsetzung dauert in der Regel nicht mehr als 2 Stunden. Das andere Verfahren benötigt i.a. eine Reaktionsdauer von 2 bis 6 Stunden, wobei der Zusatz eines Katalysators wie Triethylamin oder Dibutylzinndiacetat vorteilhaft sein kann.

Die Benzoylisocyanate (III) sind bekannte Verbindungen; man kann sie z.B. nach den Vorschriften in J. Org. Chem. 28, 1805-1811 (1963) oder J. Agr. Food Chem. 21, 348 (1973) erhalten.

Die Aminostilbene (II) sind ebenfalls bekannt oder sie können in analoger Weise wie die bekannten erhalten werden, vgl. z.B. Weygand-Hilgetag, Org. Chem. Experimentierkunst, 1970, S. 430, 570, 744, 746, 958 oder Synthesis 1979, S. 712. Das gleiche gilt für die Stilbenylisocyanate (vgl. z.B. Weygand-Hilgetag, Organisch-Chemische Experimentierkunst, 1970; DE-OS 25 38 178).

Herstellungsbeispiele

A. Unter Stickstoff werden 2,5 g Natriumhydrid (NaH; 80 %ig) in in 250 ml Tetrahydrofuran (THF) vorgelegt. Man setzt 0,1 g eines Kronenethers (15-Krone-5) zu und tropft bei 5 bis 10°C 23 g 2-Chlor-4-nitrobenzylphosphonsäurediethylester und 10 g 4-Chlorbenzaldehyd in 150 ml THF zu. Während 2 Stunden erwärmt man langsam auf Raumtemperatur, zerstört noch vorhandenes Hydrid mit 20 ml Ethanol, gießt auf 3 l Wasser, saugt ab und trocknet bei 50°C i.V. 18,3 g trans-2,4'-Dichlor-4-nitrostilben vom Fp. 138 bis 141°C.

B. 18,2 g des nach A erhaltenen Produkts werden in 150 ml THF mit 5 g Raney-Nickel im Autoklaven 15 h bei 50°C und 200 bar $H_2$-Druck hydriert. Man trennt vom Katalysator ab, engt ein und wäscht mit n-Hexan. 12 g trans-2,4'-Dichlor-4-aminostilben vom Fp. 70 bis 75°C.

C. Bei Raumtemperatur werden 5,5 g des nach B erhaltenen Produkts in 60 ml Toluol vorgelegt. Man tropft 4,2 g 2,6-Difluorbenzoylisocyanat zu, rührt über Nacht, saugt ab, wäscht mit Toluol und trocknet. Man erhält 8,2 g der Verbindung, die in der nachstehenden Tabelle als Wirkstoff 2 aufgeführt ist.

D. 250 g 2,4-Dichlorbenzyl-triphenylphosphoniumchlorid und 75 g 4-Nitrobenzaldehyd werden in 1 l Methanol gelöst; unter Rühren gibt man 37 g $NaOCH_3$ in Portionen so zu, daß die Temperatur bis auf etwa 60°C ansteigt. Nach eintägigem Rühren wird abgesaugt, mit Wasser gewaschen und getrocknet. Man erhält 124,9 g 2,4-Dichlor-4'-nitrostilben als cis/trans-Gemisch (3:7) vom Fp. 94 bis 95°C. Durch wiederholte fraktionierte Kristallisation aus Cyclohexan erhält man die reine trans-Verbindung (Fp. 130 bis 132°C) sowie 95 %ige cis-Verbindung (Fp. 93 bis 84°C).

E. 23 g trans-2,4-Dichlor-4'-nitrostilben werden in 300 ml Eisessig gelöst und mit 60 g Zinn-II-Chlorid versetzt. Man leitet HCl-Gas ein, wobei die Temperatur zunächst auf 70°C ansteigt und dann wieder fällt; unter fortwährendem Heizen wird 5 Stunden lang HCl-Gas bei

50°C eingeleitet, dann der größte Teil Eisessig abdestilliert, der Rückstand mit Eis-Natronlauge stark alkalisch gemacht und mit Ether extrahiert. Der Extrakt wird nach dem Trocknen eingeengt und liefert kristallines trans-2,4-Dichlor-4'-aminostilben (Fp. 92 bis 93°C). Aus cis-2,4-Dichlor-4'-nitrostilben wird entsprechend cis-2,4-Dichlor-4'-aminostilben als Öl erhalten.

F/G 4 g cis-2,4-Dichlorbenzyl-4'-aminostilben wird in 30 ml THF gelöst, mit 2,6-Difluorbenzoylisocyanat versetzt und nach eintägigem Stehen eingeengt. Der verbleibende Rückstand wird mit Diisopropylether verrührt und abgesaugt. Man erhält die in Tabelle 1 als Wirkstoff 6 aufgeführte Verbindung und entsprechend aus trans-2,4-Dichlorbenzyl-4'-aminostilben die Verbindung 7 der Tabelle.

Die übrigen Wirkstoffe der Tabelle werden auf entsprechende Weise aus entsprechenden Vor- bzw. Zwischenprodukten erhalten. Soweit sie physikalisch gekennzeichnet sind, wurden sie hergestellt und ihre biologische Wirkung in einem Vorverfahren getestet.

Die nicht mit physikalischen Angaben versehenen Wirkstoffe können aus entsprechenden Vorprodukten erhalten werden. Sie lassen aufgrund stuktureller Verwandtschaft eine gleichartige Wirkung erwarten wie die untersuchten Wirkstoffe.

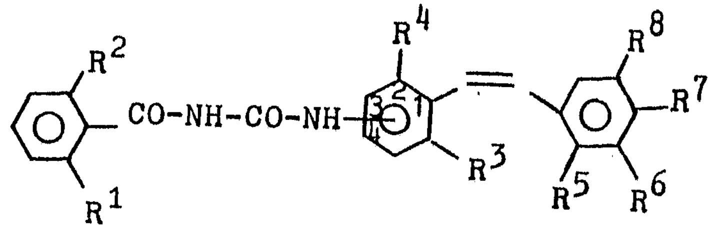

| Nr. | $R^1$ | $R^2$ | $R^3$ | $R^4$ | $R^5$ | $R^6$ | $R^7$ | $R^8$ | Pos.-NH | Isomer | Fp. °C |
|---|---|---|---|---|---|---|---|---|---|---|---|
| 1 | F | F | H | H | H | H | Cl | H | 4 | trans | 246-248 |
| 2 | F | F | Cl | H | H | H | Cl | H | 4 | trans | 225-235 |
| 3 | F | F | H | H | Cl | H | Cl | H | 3 | trans | |
| 4 | F | F | H | H | H | Cl | Cl | H | 4 | cis/trans | 175-178 |
| 5 | F | F | H | H | Cl | H | Cl | H | 4 | cis/trans | 190-195 |
| 6 | F | F | H | H | Cl | H | Cl | H | 4 | cis | 172-174 |
| 7 | F | F | H | H | Cl | H | Cl | H | 4 | trans | 227-228 |
| 8 | F | F | H | H | H | H | H | $CF_3$ | 4 | cis/trans | |
| 9 | F | F | Cl | H | Cl | H | Cl | H | 4 | cis/trans | 193-195 |
| 10 | F | F | H | H | H | H | $CH_3$ | H | 4 | cis/trans | |
| 11 | F | F | Cl | H | Cl | H | H | H | 4 | trans | |
| 12 | F | F | Cl | H | H | H | H | Cl | 4 | trans | |
| 13 | F | F | Cl | H | Cl | Cl | Cl | H | 4 | trans | |
| 14 | F | F | Cl | H | H | H | Br | H | 4 | trans | |
| 15 | F | F | Cl | H | H | H | $OC_2HF_4$ | H | 4 | trans | |
| 16 | F | F | Cl | Cl | H | H | F | H | 4 | trans | |
| 17 | F | F | Cl | Cl | H | F | H | H | 4 | trans | |
| 18 | F | F | $CF_3$ | H | H | H | Cl | H | 4 | trans | |
| 19 | F | F | Cl | H | H | H | $t-C_4H_9$ | H | 4 | trans | |
| 20 | F | F | H | H | H | H | $t-C_4H_9$ | H | 4 | trans | 235-237 |
| 21 | F | F | Br | H | H | H | H | $CF_3$ | 4 | trans | |

| Nr. | $R^1$ | $R^2$ | $R^3$ | $R^4$ | $R^5$ | $R^6$ | $R^7$ | $R^8$ | Pos.-NH | Isomer | Fp. °C |
|---|---|---|---|---|---|---|---|---|---|---|---|
| 22 | F | F | F | H | H | H | Cl | H | 4 | trans | |
| 23 | F | F | Cl | H | H | H | $OCHF_2$ | H | 4 | trans | |
| 24 | F | F | Cl | H | H | H | $OC_2HClF_3$ | H | 4 | trans | |
| 25 | Cl | H | H | H | H | H | Cl | H | 4 | cis/trans | > 270 |
| 26 | Cl | H | Cl | H | H | H | Cl | H | 4 | trans | |
| 27 | Cl | H | H | H | Cl | H | Cl | H | 3 | trans | |
| 28 | Cl | H | H | H | H | Cl | Cl | H | 4 | cis/trans | |
| 29 | Cl | H | H | H | Cl | H | Cl | H | 4 | cis/trans | |
| 30 | Cl | H | H | H | Cl | H | Cl | H | 4 | cis | 203-205 |
| 31 | Cl | H | H | H | Cl | H | Cl | H | 4 | trans | 231-232 |
| 32 | Cl | H | H | H | H | H | H | $CF_3$ | 4 | cis/trans | |
| 33 | Cl | H | Cl | H | Cl | H | Cl | H | 4 | cis-trans | 211-213 |
| 34 | Cl | H | H | H | H | H | $CH_3$ | H | 4 | cis/trans | |
| 35 | Cl | H | Cl | H | Cl | H | H | H | 4 | trans | |
| 36 | Cl | H | Cl | H | H | H | H | Cl | 4 | trans | |
| 37 | Cl | H | Cl | H | Cl | Cl | Cl | H | 4 | trans | |
| 38 | Cl | H | Cl | H | H | H | Br | H | 4 | trans | |
| 39 | Cl | H | Cl | H | H | H | $OC_2HF_4$ | H | 4 | trans | |
| 40 | Cl | H | Cl | Cl | H | H | F | H | 4 | trans | |
| 41 | Cl | H | Cl | Cl | H | F | H | H | 4 | trans | |
| 42 | Cl | H | $CF_3$ | H | H | H | Cl | H | 4 | trans | |
| 43 | Cl | H | Cl | H | H | H | $t-C_4H_9$ | H | 4 | trans | |
| 44 | Cl | H | H | H | H | H | $t-C_4H_9$ | H | 4 | trans | 248-250 |
| 45 | Cl | H | Br | H | H | H | H | $CF_3$ | 4 | trans | |

| Nr. | $R^1$ | $R^2$ | $R^3$ | $R^4$ | $R^5$ | $R^6$ | $R^7$ | $R^8$ | Pos.-NH | Isomer | Fp. °C |
|---|---|---|---|---|---|---|---|---|---|---|---|
| 46 | Cl | H | F | H | H | H | Cl | H | 4 | trans | |
| 47 | Cl | H | Cl | H | H | H | $OCHF_2$ | H | 4 | trans | |
| 48 | Cl | H | Cl | H | H | H | $OC_2HClF_4$ | H | 4 | trans | |
| 49 | Cl | Cl | H | H | H | H | Cl | H | 4 | trans | 240-244 |
| 50 | Cl | Cl | Cl | H | H | H | Cl | H | 4 | trans | 260-265 |
| 51 | Cl | Cl | H | H | Cl | H | Cl | H | 3 | trans | |
| 52 | Cl | Cl | H | H | H | Cl | Cl | H | 4 | cis/trans | |
| 53 | Cl | Cl | H | H | Cl | H | Cl | H | 4 | cis/trans | |
| 54 | Cl | Cl | H | H | Cl | H | Cl | H | 4 | cis | 173-175 |
| 55 | Cl | Cl | H | H | Cl | H | Cl | H | 4 | trans | 247-248 |
| 56 | Cl | Cl | H | H | H | H | H | $CF_3$ | 4 | cis/trans | |
| 57 | Cl | Cl | Cl | H | Cl | H | Cl | H | 4 | cis/trans | |
| 58 | Cl | Cl | H | H | H | H | $CH_3$ | H | 4 | cis/trans | |
| 59 | Cl | Cl | Cl | H | Cl | H | H | H | 4 | trans | |
| 60 | Cl | Cl | Cl | H | H | H | H | Cl | 4 | trans | |
| 61 | Cl | Cl | Cl | H | Cl | Cl | Cl | H | 4 | trans | |
| 62 | Cl | Cl | Cl | H | H | H | Br | H | 4 | trans | |
| 63 | Cl | Cl | Cl | H | H | H | $OC_2HF_4$ | H | 4 | trans | |
| 64 | Cl | Cl | Cl | Cl | H | H | F | H | 4 | trans | |
| 65 | Cl | Cl | Cl | Cl | H | F | H | H | 4 | trans | |
| 66 | Cl | Cl | $CF_3$ | H | H | H | Cl | H | 4 | trans | |
| 67 | Cl | Cl | Cl | H | H | H | $t-C_4H_9$ | H | 4 | trans | |
| 68 | Cl | Cl | H | H | H | H | $t-C_4H_9$ | H | 4 | trans | 225-228 |
| 69 | Cl | Cl | Br | H | H | H | H | $CF_3$ | 4 | trans | |
| 70 | Cl | Cl | F | H | H | H | Cl | H | 4 | trans | |

| Nr. | $R^1$ | $R^2$ | $R^3$ | $R^4$ | $R^5$ | $R^6$ | $R^7$ | $R^8$ | Pos.-NH | Isomer | Fp. °C |
|---|---|---|---|---|---|---|---|---|---|---|---|
| 71 | Cl | Cl | Cl | H | H | H | $OCHF_2$ | H | 4 | trans | |
| 72 | Cl | Cl | Cl | H | H | H | $OC_2HClF_3$ | H | 4 | trans | |
| 73 | $CH_3$ | H | H | H | H | H | Cl | H | 4 | trans | |
| 74 | $CH_3$ | H | Cl | H | H | H | Cl | H | 4 | trans | |
| 75 | Cl | F | H | H | Cl | H | Cl | H | 3 | trans | |
| 76 | Cl | F | H | H | H | Cl | Cl | H | 4 | cis/trans | |
| 77 | Cl | F | H | H | Cl | H | Cl | H | 4 | cis/trans | |
| 78 | $CH_3$ | Cl | H | H | Cl | H | Cl | H | 4 | cis | |
| 79 | $CH_3$ | Cl | H | H | Cl | H | Cl | H | 4 | trans | |
| 80 | $CH_3$ | Cl | H | H | H | H | H | $CF_3$ | 4 | cis/trans | |
| 81 | $CH_3$ | F | Cl | H | Cl | H | Cl | H | 4 | cis/trans | |
| 82 | $CH_3$ | F | H | H | H | H | $CH_3$ | H | 4 | cis/trans | |
| 83 | $CH_3$ | F | Cl | H | Cl | H | H | H | 4 | trans | |
| 84 | $CH_3$ | H | Cl | H | H | H | H | Cl | 4 | trans | |
| 85 | $CH_3$ | H | Cl | H | Cl | Cl | Cl | H | 4 | trans | |
| 86 | $CH_3$ | H | Cl | H | H | H | Br | H | 4 | trans | |
| 87 | $CH_3$ | H | Cl | H | H | H | $OC_2HF_4$ | H | 4 | trans | |
| 88 | $CH_3$ | H | Cl | Cl | H | H | F | H | 4 | trans | |
| 89 | $CH_3$ | H | Cl | Cl | H | F | H | H | 4 | trans | |
| 90 | $CH_3$ | H | $CF_3$ | H | H | H | Cl | H | 4 | trans | |
| 91 | $CH_3$ | H | Cl | H | H | H | $t-C_4H_9$ | H | 4 | trans | |
| 92 | $CH_3$ | H | Br | H | H | H | H | $CF_3$ | 4 | trans | |
| 93 | $CH_3$ | H | F | H | H | H | Cl | H | 4 | trans | |
| 94 | $CH_3$ | H | Cl | H | H | H | $OCHF_2$ | H | 4 | trans | |
| 95 | $CH_3$ | H | Cl | H | H | H | $OC_2HClF_3$ | H | 4 | trans | |

Die vorstehend aufgeführten und andere erfindungsgemäße Wirkstoffe werden auf die für N-Benzoyl-N'-phenylharnstoffe übliche Weise angewendet. Angaben zur Formulierung, Anwendungstechnik und Wirkungsweise und Angaben über geeignete Mischungspartner zur Erzielung synergistischer und anderer vorteilhafter Wirkungen können beispielsweise der US-A-4 320 122 oder der EP-A-14 674 entnommen werden.

<u>Zuchtversuch mit Maiszünsler-Larven (Ostrinia nubilalis)</u>

Die Zucht erfolgt auf einem Nährboden folgender Zusammensetzung:

| | |
|---|---|
| 515 g | Maismehl |
| 130 g | Weizenkeime |
| 137 g | Bierhefe |
| 18 g | Ascorbinsäure |
| 10 g | Cellulosepulver |
| 5 g | Nipagin |
| 20 g | Wessons Salz |
| 20 ml | Vitaminlösung |
| 80 g | Agar |
| 3100 ml | Wasser |

Alle Versuchsstufen laufen doppelt in 250 ml Bechern mit 100 ml Nährsubstrat und 10 Raupen (d.h. 20 Raupen pro Konzentration). Es ist darauf zu achten, daß die Wirkstoffaufbereitung sorgfältig untergemischt wird, um eine gleichmäßige Verteilung zu gewährleisten. Die Beobachtung erstreckt sich bis zum Schlupf der Falter.

| Ergebnis: | ppm | % Mort. |
|---|---|---|
| Verbindung Nr. 2 | 1,0 | 100 |
| | 0,4 | 80 |
| Verbindung Nr. 3 | 0,4 | 100 |
| | 0,2 | 80 |
| Verbindung Nr. 5 | 0,2 | 90 |
| Vergleichsmittel (Diflubenzuron) | 2,0 | 60 |

<u>Chemosterilisation bei Stubenfliegen (Musca domestica)</u>

Ca. 100 schlüpfreife Puppen von Stubenfliegen werden in Folienbehälter mit 8,5 l Volumen gesetzt. Sie erhalten hier 2 g Futter, bestehend aus

6 Teilen Kristallzucker
6 Teilen Trockenmilch
1 Teil Eipulver

dem zunächst 20 mg des Wirkstoffes in organischer Lösung zugefügt wurden (= 1 %).

Wasser wird in ausreichender Menge auf Zellstoff in 100 ml Kunststoffbechern geboten. Die Tiere bleiben ca. 8 Tage in diesen Käfigen bei 22°C und Tag- und Nachtrhythmus.

Am 8. Tag gibt man ein Eiablagegefäß mit in Magermilch getränktem Zellstoff in die Käfige. Hier erfolgt innerhalb der nächsten 24 Stunden die Eiablage.

Beim Einstellen der Eiablagegefäße wird die Fraßgiftwirkung anhand der toten Fliegen beurteilt.

Nach 3 - 4 Tagen wird die Entwicklung der Eier auf der Milchwatte beurteilt.

| Ergebnis: | % | % Hemmung |
|---|---|---|
| Verbindung Nr. 2 | 0,04 | 80 |
| Verbindung Nr. 3 | 0,1 | 100 |
| Verbindung Nr. 5 | 0,02 | 100 |
| Vergleichsmittel (Diflubenzuron) | 1,0 | ca. 80 |

<u>Zuchtversuch mit Blattläusen (Megoura viciae) auf Bohnen (Vicia faba)</u>

Getopfte Bohnenpflanzen, die das 1. Folgeblattpaar entwickelt haben, werden in der Spritzkabine mit der wäßrigen Wirkstoffaufbereitung tropfnaß gespritzt. Nach dem Trocknen der Beläge (ca. eine Stunde) belegt man jede Pflanze mit 10 adulten Blattläusen. Um ein Entweichen der Läuse zu verhindern, umgibt man die Pflanze mit einem Plastikzylinder (Ø 10 cm), der am oberen Ende eine Fluonbarriere trägt. Nach 7 Tagen wird die Folgegeneration an Blattläusen ausgezählt.

| Ergebnis: | % | % Mort. |
|---|---|---|
| Verbindung Nr. 2 | 0,1 | ca. 80 |
| | 0,04 | ca. 50 |
| Verbindung Nr. 3 | 0,04 | ca. 90 |
| Verbindung Nr. 5 | 0,04 | ca. 90 |
| Vergleichsmittel (Diflubenzuron) | 0,1 | 50 |

Zuchtversuch mit Kornkäfer (Sitophilus granaria)

Portionen von 100 g Roggen werden mit 200 mg Talkum-Wirkstoff gebeizt, indem man in einer Flasche beide Teile 5 Minuten lang schüttelt. Dabei bleibt fast der gesamte Staub an den Körnern kleben.

Der Roggen wird in 0,25 l Milchflaschen gefüllt und mit je 200 Kornkäfern infiziert.

Nach 14 Tagen werden die Käfer abgesiebt und die Mortalitätsrate bestimmt. Der Roggen wird in die Versuchsgefäße zurückgegeben und die Entwicklung der $F_1$-Generation abgewartet.

| Ergebnis: | ppm | % Hemmung |
|---|---|---|
| Verbindung Nr. 2 | 2 | ca. 80 |
| Verbindung Nr. 3 | 10 | ca. 90 |
| Verbindung Nr. 5 | 10 | ca. 80 |

Patentansprüche

1. N-Benzoyl-,N'-stilbenylharnstoffe der Formeln I bzw. Ia

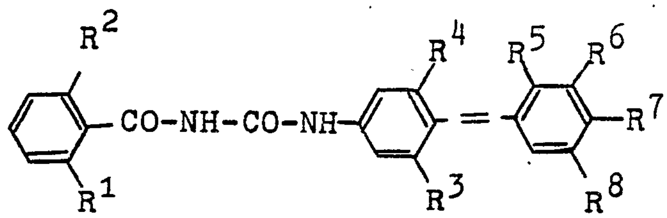

(I),

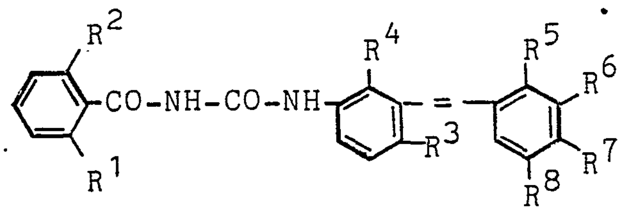

(Ia)

in denen bedeutet

$R^1$ Fluor, Chlor oder Methyl,

$R^2$ Fluor, Chlor oder Wasserstoff,

$R^3$ und $R^4$ gleiche oder verschiedene Substituenten, nämlich Wasserstoff, Chlor, Brom, Fluor oder Trifluormethyl und

$R^5$ bis $R^8$ Wasserstoff oder mindestens einen Substituenten, nämlich Fluor, Chlor, Brom, Alkyl mit 1 bis 4 C-Atomen, das mit Fluor und/oder Chlor substituiert sein kann, Alkoxi mit 1 bis 4 C--Atomen, das mit Fluor und/oder Chlor substituiert ist.

2. Verfahren zur Herstellung von N-Benzoyl-,N'-stilbenylharnstoffen gemäß Anspruch 1, dadurch gekennzeichnet, daß man ein entsprechendes Aminostilben II

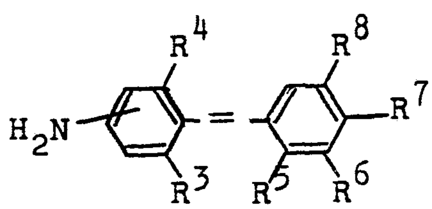

(II),

mit einem entsprechenden Benzoylisocyanat (III)

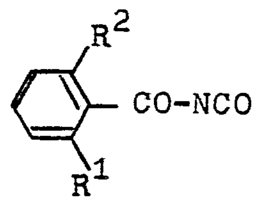
(III),

in an sich bekannter Weise umsetzt.

3. Verfahren zur Herstellung von N-Benzoyl-N'-stilbenylharnstoffen gemäß Anspruch 1, <u>dadurch gekennzeichnet</u>, daß man eine entsprechende Verbindung (IV)

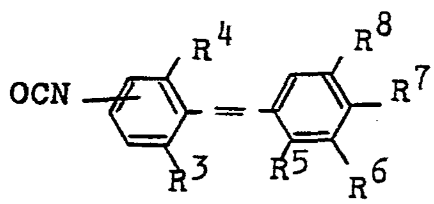
(IV),

mit einem entsprechenden Benzamid (V)

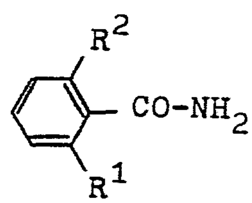
(V),

umsetzt.

4. Schädlingsbekämpfungsmittel, enthaltend einen Wirkstoff der Formel I oder Formel Ia gemäß Anspruch 1.

5. Schädlingsbekämpfungsmittel, enthaltend einen festen oder flüssigen Trägerstoff und/oder mindestens einen weiteren Wirkstoff und einen Wirkstoff der Formel I bzw. Ia gemäß Anspruch 2.

6. Verwendung von N-Benzoyl-,N'stilbenylharnstoffen der Formel I bzw. Ia gemäß Anspruch 1 bzw. von Mitteln gemäß Anspruch 4 oder 5 zur Bekämpfung von Schädlingen, insbesondere Insekten und Akariden.

7. Verfahren zur Bekämpfung von Schädlingen, insbesondere Insekten und Akariden, <u>dadurch gekennzeichnet</u>, daß man eine wirksame Menge eines N-Benzoyl-,N'-stilbenylharnstoffs der Formel I bzw. Ia gemäß Anspruch 1 auf die Schädlinge und/oder deren Lebensraum einwirken läßt.